# EUROPEAN PATENT SPECIFICATION

(11) **EP 1 581 194 B1**
(45) Date of publication and mention of the grant of the patent: **16.07.2008**
(21) Application number: 03812825.2
(22) Date of filing: 03.12.2003
(51) Int. Cl.: A61K 9/24, A61K 31/22, A61K 31/616

(54) **MULTILAYERED TABLET CONTAINING PRAVASTATIN AND ASPIRIN AND METHOD**
MEHRSCHICHTIGE TABLETTE MIT PRAVASTATIN UND ASPIRIN UND VERFAHREN
COMPRIMÉ MULTICOUCHE CONTENANT DE LA PRAVASTATINE ET DE L'ASPIRINE ET PROCÉDÉ

(30) Priority: 11.12.2002 US 316424
(43) Date of publication of application: 05.10.2005
(73) Proprietor: Bristol-Myers Squibb Company, Princeton, NJ 08543-4000 (US)
(72) Inventor: BENKERROUR, Loutfy, F-75015 Paris (FR); GALLEY, Olivier, F-44380 Pornichet (FR); QUINET, Francoise, F-44300 Nantes (FR); TIMMINS, Peter, Nocturum, Prenton, Merseyside CH43 9HJ (GB); ABEBE, Admassu, F-44700 Orvault (FR)
(74) Representative: Vossius & Partner
(86) International application number: PCT/US2003/038774
(87) International publication number: WO 2004/052289

(56) References cited:
- WO-A-94/06416
- WO-A-03/020243
- US-A- 4 590 183
- US-B1- 6 235 311
- US-B1- 6 372 255
- US-B1- 6 475 521

## Description

The present invention relates to a pharmaceutical composition in the form of a tablet having three or more layers which includes aspirin in one layer and pravastatin in a second layer, which layers are separated from each other by at least one middle barrier layer which includes one or more buffering agents, in a manner to minimize interaction of aspirin with the statin, for use in lowering cholesterol and reducing risk of a myocardial infarction.

### BACKGROUND OF THE INVENTION

The use of aspirin for reducing the risk of a myocardial infarction and the use of statins for lowering cholesterol and preventing or treating atherosclerosis and cardiovascular disease and cerebrovascular disease are well documented. In fact, it is not uncommon that patients having elevated cholesterol levels who are at high risk for a myocardial infarction take both a statin and aspirin. However, use of both a statin and aspirin may require special care to insure that drug interaction, including physical and chemical incompatibility, and side effects, are kept to a minimum while achieving maximum benefit from these drugs.

With regard to possible physical drug interaction, aspirin is an acid, while some of the statins, such as pravastatin, atorvastatin and cerivastatin, are alkali salts. Thus, mixing of such statins (alkali salts) with aspirin could result in aspirin hydrolysis as well as statin degradation. Pravastatin is an acid labile compound. When pravastatin and aspirin are combined, the aspirin could cause pravastatin degradation which could result in reduced bioavailability of pravastatin.

Aspirin is known for causing gastrointestinal irritation and possibly bleeding when used for long-term therapy. It is therefore desirable in long-term aspirin therapy that the aspirin be provided in a form which minimizes side effects, for example a buffered aspirin formulation.

In view of the above, it is seen that there is a long-felt want in patients required to take both a statin and aspirin for a statin-aspirin formulation which provides for maximum cholesterol lowering and reduction of risk of a myocardial infarction without the undesirable side effects and drug interaction normally associated with use of such combination.

U.S. Patent No. 6,235,311 to Ullah et al. discloses a bilayered tablet which includes aspirin in a first layer and a statin in a second layer, which tablet is formulated to minimize interaction between aspirin and the statin. The layer containing the statin includes one or more buffering agents to inhibit undesirable statin/aspirin interaction.

WO94/06416 discloses a three-layered tablet which includes an outer immediate release layer, an outer slow release layer, and a middle barrier layer, which may contain a buffer, which separates the immediate release layer from the slow release layer. Among the drugs which may be present in the tablets are non-steroidal anti-inflammatory agents.

U.S. Patent No. 4,590,183 to Bailey discloses a multi-layered tablet which contains aspirin and sodium thiosulfate which may be separated from each other by an inert barrier.

WO03/020 243 relates to pharmaceutical formulations for treating patients at elevated cardiovascular risk, and more particularly to dosage forms that combine a cholesterol-lowering agent, an inhibitor of the renin-angiotensin system, and aspirin.

### DESCRIPTION OF THE INVENTION

In accordance with the present invention, a pharmaceutical composition is provided which is preferably in the form of a tablet containing pravastatin and aspirin formulated to reduce and/or inhibit pravastatin:aspirin interaction. The tablet of the invention is in the form of a sandwich structure which includes one layer containing aspirin, another layer containing pravastatin, and a middle barrier layer separating said first layer containing aspirin from said other layer containing pravastatin, wherein the middle barrier layer includes one or more buffering agents. Optionally, the tablet may include an aspirin layer and a pravastatin layer separated by an intermediate or middle barrier layer with an additional layer or layers applied to the outer face of the pravastatin layer, the aspirin layer or both. These additional layers can be for aesthetic purposes, to disguise the taste of the drug substances, and/or to provide for delivery of additional active materials, such as buffers or other ingredients such as niacin, which are often co-administered with statins.

The tablet of the invention provides for maximum patient benefits including maximum cholesterol lowering and reduced risk of a myocardial infarction with minimal physical and chemical incompatibility (including minimal pravastatin:aspirin interaction), and reduced side effects normally associated with use of such drugs.

In addition, in accordance with the present invention, a pharmaceutical composition containing a combination of pravastatin and aspirin in a single dosage form, as described above, in a manner so as to minimize interaction of the pravastatin and aspirin is provided for lowering serum cholesterol, preventing or inhibiting or treating atherosclerosis, and/or reducing risk of or treating a cardiovascular event or disease including coronary artery disease and cerebrovascular disease, wherein the pharmaceutical composition is to be administered to a patient in need of treatment.

Preferred pharmaceutical compositions of the present invention may take the form of several different embodiments. Thus, in one embodiment of the present invention, a pharmaceutical composition is provided as a trilayered sandwich structure wherein the pravastatin and aspirin are formulated together in a single tablet. The tablet of the invention is preferably in the form of a trilayered tablet which includes a first layer, a second layer in the form of a middle barrier layer and a third layer. Aspirin, in the form of granules or crystals of a defined size will be present in the first layer together with optional excipients as described hereinafter, while the pravastatin will be present in the third layer. The middle barrier layer includes one or more buffering agents (as necessary to reduce, inhibit or prevent undesirable statin/aspirin interaction) and optionally one or more excipients as described hereinafter.

In addition, the trilayered tablet of the invention may include an outer protective coating or finishing layer covering the first layer and/or third layer as described hereinafter.

In addition, in accordance with the present invention, a pharmaceutical composition is provided which is in the form of a trilayered tablet as described above which includes in one outer layer aspirin granules having an enteric coating to provide maximum efficacy while minimizing side effects resulting from prolonged aspirin therapy.

Another embodiment of the pharmaceutical composition of the invention includes granules of enteric coated aspirin in the first layer and enteric coated pravastatin in the third layer.

The tablets containing the enteric coated granules of aspirin and statin may also include an outer protective coating or finishing layer.

### DETAILED DESCRIPTION OF THE INVENTION

The pharmaceutical composition of the invention which includes a combination of pravastatin and aspirin is effective in preventing, reducing and/or treating elevated cholesterol levels (such as in hypercholesterolemia), atherosclerosis, cardiovascular events and disease including coronary events and cerebrovascular events, and coronary artery disease and/or cerebrovascular disease.

The terms "cardiovascular event(s)" and "cardiovascular disease" as employed herein refer to coronary and/or cerebrovascular event(s) and disease including primary myocardial infarction, secondary myocardial infarction, myocardial ischemia, angina pectoris (including unstable angina), congestive heart failure, sudden cardiac death, cerebral infarction, cerebral thrombosis, cerebral ischemia, transient ischemic attack and the like.

The term "coronary artery disease" (CAD) as employed herein refers to diseases including atherosclerosis of the coronary arteries, previous myocardial infarction, ischemia, angina pectoris and/or heart failure.

The term "cerebrovascular disease" as employed herein refers to diseases including atherosclerosis of the intracranial and/or extracranial arteries, cerebral infarction, cerebral thrombosis, cerebral ischemia, stroke, and/or transient ischemic attacks.

The term "pravastatin" as employed herein refers to pravastatin, pravastatin sodium, other pravastatin salts, and all forms of pravastatin including the dihydroxy acid form and the lactone form.

Aspirin will preferably be employed in the form of acetylsalicylic acid also referred to as salicylic acid acetate, or its salts, esters or complexes.

The pharmaceutical composition of the invention in the form of a tablet will include aspirin in amounts from about 10 to about 800 mg, preferably from about 25 to about 650 mg, most preferably from about 50 to about 325mg.

The aspirin for use in forming the pharmaceutical composition of the invention will preferably be in the form of granules having an average particle size within the range from about 10 microns to about 2 mm, more preferably from about 50 microns to about 1.0 mm.

The pharmaceutical composition of the invention will contain pravastatin in an amount as normally employed as exemplified in the 56^{th} Edition of the Physician's Desk Reference (PDR) (2002). Thus, pravastatin may be employed in amounts within the range from about 0.1 mg to 2000 mg per day in single or divided doses, and preferably from about 0.2 to about 200 mg per day, and most preferably, a daily dosage of 10 to 160 mg may be employed in single or divided doses.

In forming the pharmaceutical composition of the invention in the form of a trilayered tablet, the first layer containing aspirin will optionally, but preferably, include bulking agents such as lactose, microcrystalline cellulose, wood cellulose, corn starch, modified corn starch, calcium phosphate, sugar, dextrose, mannitol, sorbitol or mixtures of two or more thereof. The bulking agent will be present in an amount from about 1 to about 90%, preferably from about 5 to about 85% by weight of the first layer containing aspirin.

The first layer may also optionally include a tabletting lubricant, such as zinc stearate, magnesium stearate, calcium stearate, talc, carnauba wax, stearic acid, palmitic acid or hydrogenated vegetable oils and fats, in an amount within the range from about 0.1 to about 4%, and preferably 0.2 to about 2% by weight of the first layer. The aspirin layer may also optionally include a disintegrant such as corn starch, potato starch, pre-gelatinised starch, crospovidone, croscarmellose sodium or sodium starch glycollate in an amount within the range from about 0.5 to about 20% by weight of the layer, and preferably from about 1 to about 10% by weight of the layer.

The third layer of the trilayered tablet containing pravastatin cholesterol lowering agent will optionally include a bulking agent such as lactose, microcrystalline cellulose, modified corn starch, calcium phosphate or other bulking agent as set out above for the first layer, in an amount within the range from about 1 to about 90%, preferably from about 5 to about 85% by weight of the third layer. In addition, the third layer may optionally include a binder such as corn starch, pregelatinized starch, polyvinyl pyrrolidone (PVP), hydroxypropylmethyl cellulose (HPMC), ethyl cellulose, cellulose acetate and the like, in an amount within the range from about 0.5 to about 20%, preferably from about 1 to about 10% by weight or the third layer, a disintegrating agent, such as croscarmellose sodium, crospovidone, pre-gelatinised starch, corn starch or sodium starch glycollate in an amount within the range from about 0.5 to about 20% by weight, preferably from about 1 to about 15% by weight of the third layer, and a tabletting lubricant such as magnesium stearate, zinc stearate, or other lubricant as set out above with respect to the first layer in an amount from about 0.1 to about 4%, preferably from about 0.2 to about 2% by weight of the third layer.

The third layer containing pravastatin may also optionally include one or more buffering agents which include conventional acid buffers such as calcium carbonate, magnesium oxide, magnesium carbonate, magnesium hydroxide, aluminum hydroxide, dihydroxyaluminum sodium carbonate, aluminum magnesium hydroxide sulfate or aluminum hydroxide magnesium carbonate co-dried gel, or any of the acid buffers set out for use in the middle barrier layer, or mixtures of one or more thereof, in amounts as needed to insure that the aspirin will be sufficiently buffered to inhibit GI side effects. Thus, amounts of buffering agent within the range from about 10 to about 800 mg, preferably from about 70 to about 300 mg will be employed depending upon the amount of aspirin present in the first layer.

The middle or barrier layer will optionally include a bulking agent such as any of the bulking agents set out above for use in the first (aspirin) layer or third (pravastatin) layer, such as microcrystalline cellulose, corn starch, lactose, and the like, in an amount within the range from about 0 to about 90%, preferably from about 5 to about 85% based on the weight of the middle barrier layer; a disintegrating agent such as any of the disintegrating agents set out above with respect to the third (pravastatin) layer in an amount with the range from about 0.5 to about 20%, preferably from about 1 to about 15% by weight of the middle barrier layer; a tabletting lubricant, such as any of the tabletting lubricants set out above with respect to the first layer and the third layer, in an amount within the range from about 0.1 to about 4%, preferably from about 0.2 to about 2% by weight of the middle barrier layer.

The middle barrier layer includes a buffering agent in an amount sufficient to reduce gastric irritation that can be caused by aspirin and insure that the aspirin will be sufficiently buffered to minimize interaction between the aspirin and pravastatin. Thus, amounts of buffering agent within the range from about 10 to about 800 mg, preferably from about 70 to about 300 mg, depending upon the amount of aspirin present in the first layer.

Examples of buffering agent suitable for use in the middle barrier layer include calcium carbonate, magnesium oxide, magnesium carbonate, magnesium hydroxide, calcium hydroxide, sodium phosphate, aluminum hydroxide, dihydroxyaluminum sodium carbonate, sodium carbonate, sodium bicarbonate, aluminum magnesium hydroxide sulfate or aluminum hydroxide, magnesium carbonate co-dried gel, sodium acetate, sodium citrate, sodium tartrate, sodium fumarate, sodium malate, sodium succinate, aluminum oxide, or mixtures of two or more thereof.

Preferred trilayered tablet formulations of the invention are set out below.
I. One to three trilayered tablet(s) containing pravastatin-aspirin where the middle barrier layer includes one or more buffering agents (Total Tablet(s) Weight = 350 to 4000 mg)

| Ingredient | Weight % (based on each separate layer) | Amount per One to Three Tablet(s) (mg) |
|---|---|---|
| First Layer containing Aspirin | | |
| Aspirin | 18 to 90 | 50 to 325 |
| Bulking Agents | 5 to 85 | 5 to 1100 |
| Optional Disintegrants | 0 to 10 | 0 to 50 |
| Binders | 0 to 50 | 0 to 250 |
| Lubricants | 0.2 to 10 | 0.18 to 50 |
| Other Excipients | 0 to 10 | 0 to 50 |
| Total Weight of First Layer | 100% | 90 to 2000 mg |

| Middle Barrier Layer containing Buffering Agents | | |
|---|---|---|
| Buffering Agents | 10 to 90 | 70 to 315 |
| Bulking Agents | 5 to 85 | 7.5 to 298 |
| Binders | 0 to 10 | 0 to 35 |
| Lubricants | 0.2 to 2 | 0.12 to 7.5 |
| Other Excipients | 0 to 10 | 0 to 35 |
| Total Weight of Middle Barrier Layer | 100% | 60 to 700 mg |

| Third Layer containing Pravastatin | | |
|---|---|---|
| Pravastatin Sodium | 5 to 40 | 10 to 160 |
| Bulking Agents | 5 to 85 | 10 to 340 |
| Binders | 1 to 10 | 2 to 40 |
| Disintegrating Agents | 1 to 15 | 2 to 60 |
| Buffering Agents | 35 to 75 | 70 to 300 |
| Lubricants | 0.2 to 2 | 0.4 to 8 |
| Other Excipients | 0 to 10 | 0 to 40 |
| Total Weight of Third Layer | 100% | 200 to 1100 mg |

Reference-Example II. One to three trilayered tablet(s) containing pravastatin-aspirin in a formulation where the middle barrier layer does not include a buffering agent (total tablet(s) weight = 350 to 4000 mg)

| Ingredient | Weight % (based on each separate layer) | Amount per One to Three Tablet(s) (mg) |
|---|---|---|
| First Layer containing Aspirin | | |
| Aspirin | 18 to 90 | 50 to 325 |
| Bulking Agents | 5 to 85 | 5 to 1100 |
| Optional Binders | 0 to 25 | 0 to 250 |
| Lubricants | 0.1 to 2 | 0.6 to 9 |
| Other Excipients | 0 to 10 | 0 to 50 |
| Total Weight of First Layer | 100% | 90 to 500 mg |

| Middle Barrier Layer (No Buffering Agents) | | |
|---|---|---|
| Bulking Agents | 5 to 85 | 3 to 255 |
| Disintegrating Agents | 0 to 10 | 0 to 30 |
| Lubricants | 0.2 to 2 | 0.2 to 6 |
| Other Excipients | 0 to 10 | 0 to 30 |
| Total Weight of Middle Barrier Layer | 100% | 60 to 350 mg |

| Third Layer containing Pravastatin | | |
|---|---|---|
| Pravastatin Sodium | 5 to 33 | 10 to 200 |
| Bulking Agents | 5 to 85 | 10 to 700 |
| Binders | 0.2 to 4 | 0.4 to 9 |
| Disintegrating Agents | 0.5 to 15 | 2 to 60 |
| Buffering Agents | 35 to 75 | 70 to 450 |
| Lubricants | 0.2 to 2 | 0.4 to 12 |
| Other Excipients | 0 to 10 | 0 to 60 |
| Total Weight of Third Layer | 100% | 200 to 1100 mg |

Specific preferred trilayered tablet formulations of the invention are set out below.
I. One to three trilayered tablet(s) containing pravastatin-aspirin where the middle barrier layer includes one or more buffering agents. 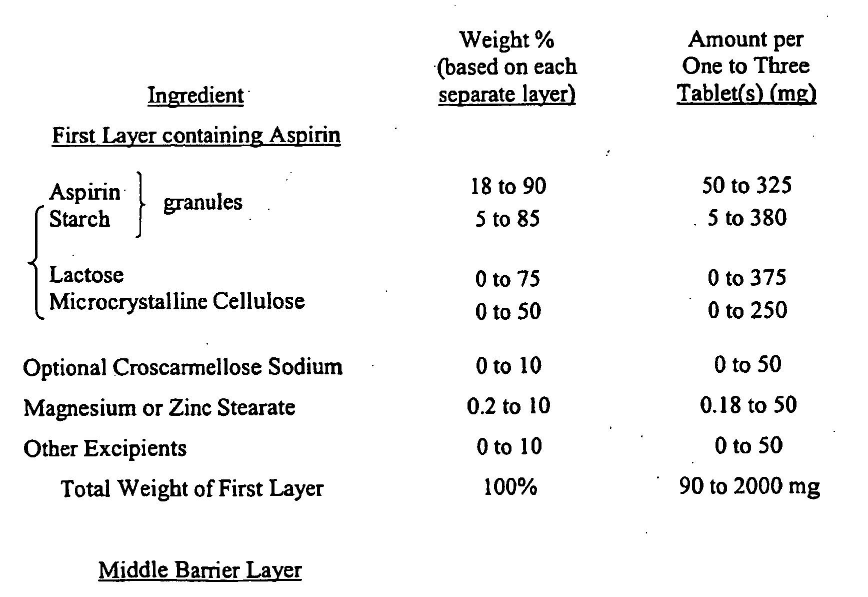 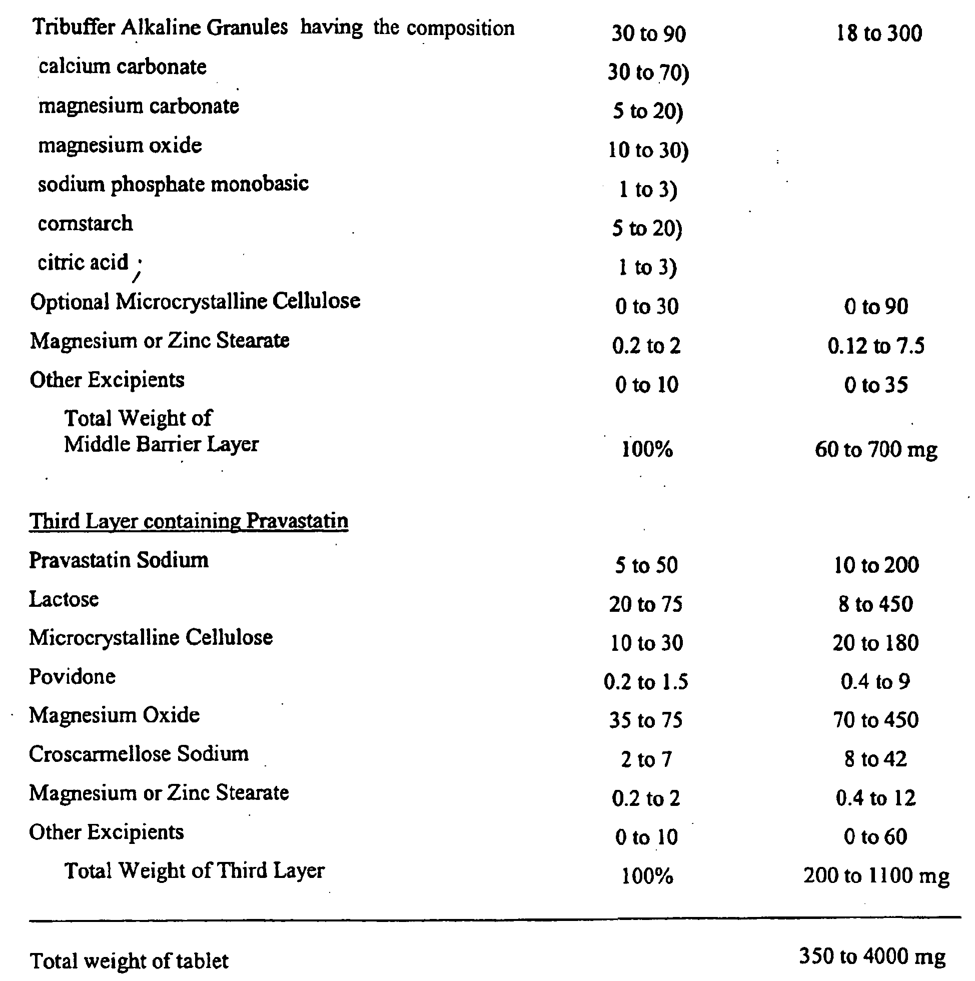
Reference-Example II. One to three trilayered tablet(s) containing pravastatin-aspirin in a formulation where the middle barrier layer does not include a buffering agent.

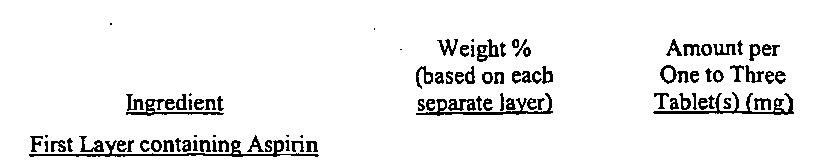

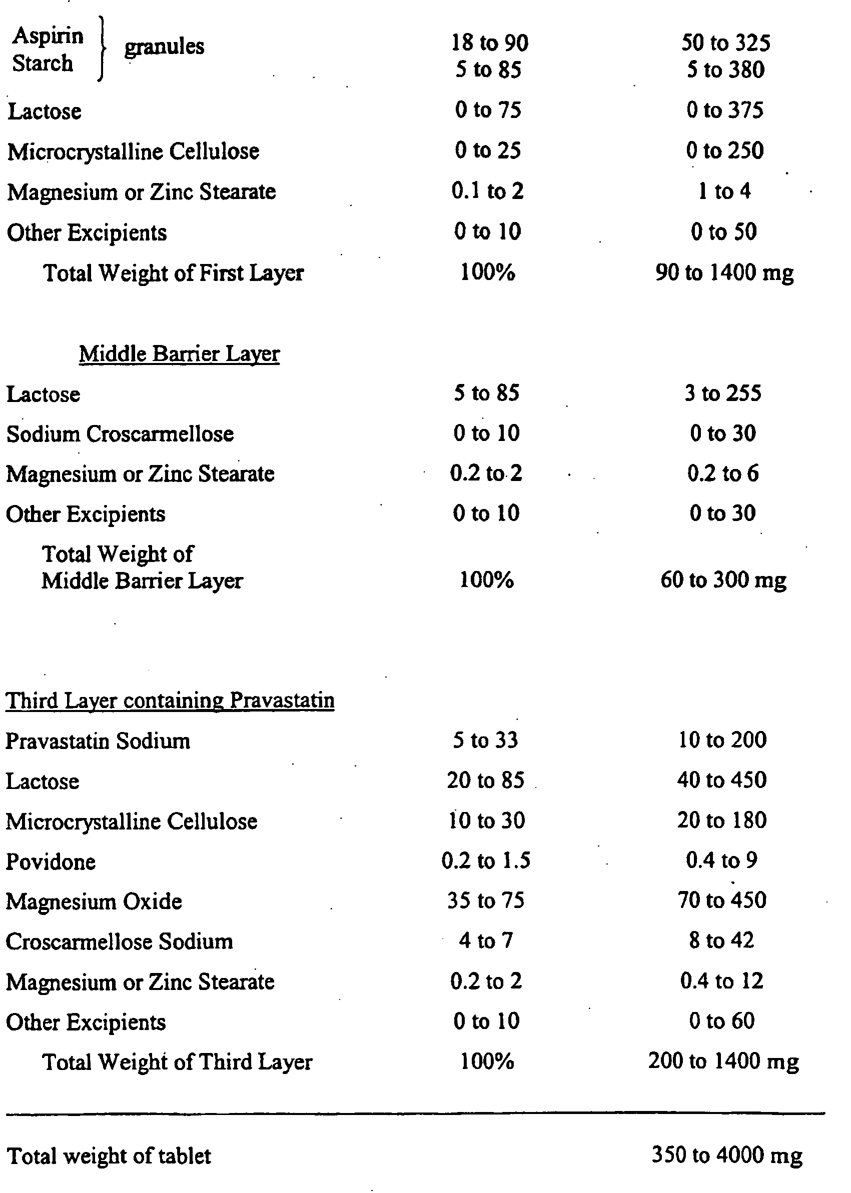

In forming a trilayered tablet of the invention, the first layer containing aspirin, the third layer containing pravastatin and the middle barrier layer may each be prepared by conventional wet granulation, dry granulation (compaction) or dry blending techniques.

The first, middle and third layers may then be compressed and combined to form a trilayered tablet employing conventional trilayer tabletting equipment.

Other conventional ingredients which may optionally be present in any of the three layers include preservatives, stabilizers, anti-adherents or silica flow conditioners or glidants, such as Syloid brand silicon dioxide as well as antioxidants such as Vitamin E, Vitamin C, and folic acid, Vitamin B₆ and Vitamin B₁₂.

The trilayer tablet of the invention may also include an outer protective coating layer which may include up to about 15% by weight of the trilayer tablet. The outer protective coating layer which is applied over the trilayered tablet may comprise any conventional coating formulations and will include one or more film-formers, such as a hydrophilic polymer like hydroxypropylmethyl cellulose (HPMC) and a hydrophobic polymer like ethyl cellulose, cellulose acetate, polyvinyl alcohol-maleic anhydride copolymers, acrylic copolymers, β-pinene polymers, glyceryl esters of wood resins and the like, and one or more plasticizers, such as polyethylene glycol, triethyl citrate, diethyl phthalate, propylene glycol, glycerin, butyl phthalate, castor oil and the like.

The film formers are applied from a solvent system containing one or more solvents including water, alcohols like methyl alcohol, ethyl alcohol or isopropyl alcohol, ketones like acetone, or ethylmethyl ketone, chlorinated hydrocarbons like methylene chloride, dichloroethane, and 1,1,1-trichloroethane.

Another embodiment of the pharmaceutical composition of the invention is formed of trilayered tablets containing enteric coated aspirin granules in the first layer.

The aspirin granules can be coated with conventional enteric polymers coatings in aqueous or non-aqueous systems. For example, Eudragit L-30D-55 (acrylic acid copolymers-Rohm Pharma) (5 to 25% solids) containing 10 to 15% of diethylphthlate (w/w) as plasticizer can be used in an aqueous system.

Other conventional enteric polymer coating systems may be employed such as Eudragit R and S series resins, (acrylic acid copolymers-Rohm Pharma), cellulose acetate phthalate, cellulose acetate maleate, cellulose acetate succinate, hydroxypropylmethyl cellulose phthalate, hydroxypropylmethylcellulose acetate succinate, and the like, and a suitable plasticizer such as triethyl citrate, diethyl phthalate, tributyl citrate, triacetin, dibutyl phthalate, dibutyl sebicate, Myvacet 940, and other commonly used plasticizers as may be suitable for particular enteric polymers can be used. It will be appreciated that any polymer with suitable plasticizer can be used in aqueous or non-aqueous system to form an enteric coating on the aspirin granule or particle.

In another embodiment of the pharmaceutical composition of the invention, the enteric coated aspirin granules described above may be further coated with an outer protective finishing coat or layer as described hereinbefore.

The double coated aspirin granules can be tableted as described above.

In yet another embodiment of the pharmaceutical composition of the invention, aspirin is enteric coated as described above and the pravastatin can optionally be enteric coated. Pravastatin can be coated in the form of pure drug or after spheronization or agglomeration. The particles for coating do not need to be perfectly spherical. These could be rods or irregular particles.

In carrying out the method of the present invention, the pharmaceutical composition of the invention containing the combination of the pravastatin and aspirin may be administered to mammalian species, such as monkeys, dogs, cats, rats, humans, etc., and, as described hereinbefore, may be incorporated in a trilayered tablet. The above dosage forms will also include the necessary carrier material, excipient, lubricant, buffer, antibacterial, bulking agent (such as mannitol), antioxidants such as Vitamin C and Vitamin E, as well as Vitamin B₆, Vitamin B₁₂, folic acid, sodium bisulfite, and the like.

The dose administered must be adjusted according to age, weight and condition of the patient, as well as the route of administration, dosage form and regimen and the desired result.

The compositions described above may be administered in the dosage forms as described above in single or divided doses of one to four times daily. It may be advisable to start a patient on a low dose combination and work up gradually to a high dose combination.

Tablets of various sizes can be prepared, e.g., up to about 1500 mg in total weight, containing the active substances in the ranges described above. These tablets can, of course, be scored to provide for fractional doses in some cases.

Some of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for cardiovascular events and disease including coronary artery disease and/or cerebrovascular disease remains or the symptoms continue. Sustained release forms of such formulations which may provide such amounts daily, biweekly, weekly, monthly and the like may also be employed. A dosing period of at least 10 days are required to achieve minimal benefit.

### EXAMPLES

The following Examples represent preferred embodiments of the present invention.

Formulations suitable for oral administration are prepared as described below.

### EXAMPLE 1

A trilayered tablet containing 81 mg aspirin in a first outer layer and 40 mg pravastatin sodium in a second outer layer with a buffered intermediate layer may be prepared as follows.

| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| Purified water | q.s.¹ |
| **Total first layer** | **400.00** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules³ | 90.00 mg in total (81 mg aspirin) |
| Microcrystalline cellulose | 48.00 |
| Lactose | 160.50 |
| Zinc stearate | 1.50 |
| **Total third layer** | **300.00** |
| **Total tablet weight** | **900.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A wet granulation process is used to prepare pravastatin granules, dissolving the pravastatin sodium and the povidone in purified water and using this solution to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The three layer tablets are prepared by having the granules of powder mixes for each layer in separate hoppers on a tablet press designed for the manufacture of layered tablets. Operating the tablet press results in triple layers tablets containing 81 mg of aspirin and 40 mg of pravastatin sodium possessing an intermediate layer containing alkaline earth buffers.

### EXAMPLE 2

A trilayered tablet containing 325 mg aspirin in a first outer layer and 40 mg pravastatin sodium in a second outer layer and an intermediate layer containing buffering agents may be prepared as follows.

| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Iron oxide red | 0.80 |
| Zinc stearate | 2.00 |
| Purified water¹ | q.s. |
| **Total first layer** | **400.00** |
| Tribuffer alkaline granules² | 199.00 |
| Zinc stearate | 1.00 |
| **Total second, intermediate layer** | **200.00** |
| **Ingredient** | **Amount per tablet (mg)** |
| Aspirin 10% starch granules³ | 361.10 mg in total (325 mg aspirin) |
| Zinc stearate | 0.90 |
| **Total third layer** | **362.00** |
| **Total tablet weight** | **962.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

The components of the three layers are processed as per example 1 and the granules transferred to a rotary tablet press designed for making layered tablets which is operated to yield tablets containing 325 mg of aspirin and 40 mg of pravastatin sodium with an intermediate layer containing alkaline earth buffers.

### EXAMPLE 3

A trilayered tablet containing 81 mg aspirin in a first outer layer and 80 mg pravastatin sodium in a second outer layer with a buffered intermediate layer may be prepared as follows.

| **Ingredient** | **Amount per tablet (mg)** |
|---|---|
| Pravastatin sodium | 80.00 |
| Lactose monohydrate | 211.80 |
| Microcrystalline cellulose | 60.20 |
| Povidone | 4.00 |
| Magnesium oxide, heavy | 28.00 |
| Croscarmellose sodium | 20.00 |
| Blue #2 aluminum lake 11-14% | 0.80 |
| Zinc stearate | 2.00 |
| Purified water | q.s.¹ |
| **Total first layer** | **406.80** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules³ | 90.00 mg in total (81 mg aspirin) |
| Microcrystalline cellulose | 48.00 |
| Lactose | 160.50 |
| Zinc stearate | 1.50 |
| **Total third layer** | **300.00** |
| **Total tablet weight** | **906.80** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A wet granulation process is used to prepare pravastatin granules, dissolving the pravastatin sodium and the povidone in purified water and using this solution to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The three layer tablets are prepared by having the granules of powder mixes for each layer in separate hoppers on a tablet press designed for the manufacture of layered tablets. Operating the tablet press results in triple layers tablets containing 81 mg of aspirin and 80 mg of pravastatin sodium possessing an intermediate layer containing alkaline earth buffers.

### EXAMPLE 4

A trilayered tablet containing 325 mg aspirin in a first outer layer and 80 mg pravastatin sodium in a second outer layer and an intermediate layer containing buffering agents may be prepared as follows.

| **Ingredient** | **Amount per tablet (mg)** |
|---|---|
| Pravastatin sodium | 80.00 |
| Lactose monohydrate | 212.60 |
| Microcrystalline cellulose | 60.20 |
| Povidone | 4.00 |
| Magnesium oxide, heavy | 28.00 |
| Croscarmellose sodium | 20.00 |
| Zinc stearate | 2.00 |
| Purified water¹ | q.s. |
| **Total first layer** | **406.80** |
| Tribuffer alkaline granules² | 199.00 |
| Zinc stearate | 1.00 |
| Total second, intermediate layer | 200.00 |
| Aspirin 10% starch granules³ | 361.10 mg in total (325 mg aspirin) |
| Zinc stearate | 0.90 |
| **Total third layer** | **362.00** |
| **Total tablet weight** | **968.80** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

The components of the three layers are processed as per Example 1 and the granules transferred to a rotary tablet press designed for making layered tablets which is operated to yield tablets containing 325 mg of aspirin and 80 mg of pravastatin sodium with an intermediate layer containing alkaline earth buffers.

### EXAMPLE 5

A trilayered tablet containing 81 mg aspirin in a first outer layer and 40 mg pravastatin sodium in a second outer layer and an intermediate layer containing alkaline earth buffering agents may be prepared as follows.

| **Ingredient** | **Amount per tablet (mg)** |
|---|---|
| Pravastatin sodium | 40.00 |
| Lactose monohydrate | 263.00 |
| Microcrystalline cellulose | 60.70 |
| Povidone | 4.00 |
| Magnesium oxide, heavy | 13.30 |
| Croscarmellose sodium | 20.20 |
| Ferric oxide yellow | 0.80 |
| Zinc stearate | 3.00 |
| Purified water | q.s.¹ |
| **Total first layer** | **405.00** |
| Tribuffer alkaline granules² | 199.00 |
| Magnesium stearate | 1.00 |
| **Total second (intermediate) layer** | **200.00** |
| Aspirin 10% starch granules³ | 90.08 mg in total (81 mg aspirin) |
| Croscannellose sodium | 8.85 |
| Lactose | 193.86 |
| Zinc stearate | 2.21 |
| **Total third layer** | **295.00** |
| **Total tablet weight** | **900.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

The components of the three layers are processed as per example 1 and the granules transferred to a rotary tablet press designed for making layered tablets which is operated to yield tablets containing 81 mg of aspirin and 80 mg of pravastatin sodium with an intermediate layer containing alkaline earth buffers.

### EXAMPLE 6

A trilayered tablet containing 81 mg aspirin in a first outer layer and 40 mg pravastatin sodium in a second outer layer and an intermediate layer containing alkaline earth buffering agents may be prepared as follows.

| **Ingredient** | **Amount per tablet (mg)** |
|---|---|
| Pravastatin sodium | 40.00 |
| Lactose monohydrate | 259.80 |
| Microcrystall-ine cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| Purified water | q.s.¹ |
| **Total first layer** | **400.00** |
| Tribuffer alkaline granules² | 198.84 |
| Brown dye PB-1596 | 0.16 |
| Magnesium stearate | 1.00 |
| **Total second (intermediate) layer** | **200.00** |
| Aspirin 10% starch granules³ | 89.55 mg in total (81 mg aspirin) |
| Lactose Fast-Flo | 160.95 |
| Microcrystalline cellulose | 48.00 |
| Zinc stearate | 1.50 |
| **Total third layer** | **300.00** |
| **Total tablet weight** | **900.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A wet granulation process is used to prepare pravastatin granules; the pravastatin sodium and the povidone are dissolved in purified water and this solution is used to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The triple layer tablets are prepared by having the granules of powder mixes for each layer in separate hoppers on a tablet press designed for the manufacture of layered tablets. Operating the tablet press results in triple layers tablets containing 81 mg of aspirin and 40 mg of pravastatin sodium possessing an intermediate layer containing alkaline earth buffers.

### EXAMPLE 7

A trilayered tablet containing 81 mg aspirin in a first outer layer and 40 mg pravastatin sodium in a second outer layer and an intermediate layer containing alkaline earth buffering agents may be prepared as follows.

| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| **Total first layer** | **400.00** |
| Tribuffer alkaline granules¹ | 198.84 |
| Brown dye PB-1596 | 0.16 |
| Magnesium stearate | 1.00 |
| Total second (intermediate) layer | 200.00 |
| Aspirin 10% starch granules³ | 89.55 mg in total (81 mg aspirin) |
| Lactose Fast-Flo | 160.95 |
| Microcrystalline cellulose | 48.00 |
| Zinc stearate | 1.50 |
| **Total third layer** | **300.00** |
| **Total tablet weight** | **900.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A direct compression process is used to prepare the pravastatin layer by blending the pravastatin sodium and the other first layer ingredients except for the zinc stearate in a suitable mixer until uniform. This powder blend is then lubricated by further mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The triple layer tablets are prepared by having the granules of powder mixes for each layer in separate hoppers on a tablet press designed for the manufacture of layered tablets. Operating the tablet press results in triple layered tablets containing 81 mg of aspirin and 40 mg of pravastatin sodium possessing an intermediate layer containing alkaline earth buffers.

### EXAMPLE 8

A trilayered tablet containing 81 mg aspirin in a first outer layer and 20 mg pravastatin sodium in a second outer layer (made by a wet granulation process) and an intermediate layer containing alkaline earth buffering agents may be prepared as follows.

| **Ingredient** | **Amount per tablet (mg)** |
|---|---|
| Pravastatin sodium | 20.00 |
| Lactose monohydrate | 129.60 |
| Microcrystalline cellulose | 30.10 |
| Povidone | 2.00 |
| Magnesium oxide, heavy | 6.60 |
| Croscarmellose sodium | 10.00 |
| Ferric oxide yellow | 0.40 |
| Zinc stearate | 1.00 |
| Purified water | q.s.¹ |
| **Total first layer** | **200.00** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules³ | 90.00 mg in total (81 mg aspirin) |
| Lactose Fast-Flo | 160.50 |
| Microcrystalline cellulose | 48.00 |
| Zinc stearate | 1.50 |
| **Total third layer** | **300.00** |
| **Total tablet weight** | **700.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A wet granulation process is used to prepare pravastatin granules, dissolving the pravastatin sodium and the povidone in purified water and using this solution to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The triple layer tablets are prepared by having the granules of powder mixes for each layer in separate hoppers on a tablet press designed for the manufacture of layered tablets. Operating the tablet press results in triple layers tablets containing 81 mg of aspirin and 20 mg of pravastatin sodium possessing an intermediate layer containing alkaline earth buffers

### EXAMPLE 9

A trilayered tablet containing 325 mg aspirin in a first outer layer and 20 mg pravastatin sodium in a second outer layer (made by a wet granulation process) and an intermediate layer containing alkaline earth buffering agents may be prepared as follows.

| **Ingredient** | **Amount per tablet (mg)** |
|---|---|
| Pravastatin sodium | 20.00 |
| Lactose monohydrate | 129.00 |
| Microcrystalline cellulose | 30.10 |
| Povidone | 2.00 |
| Magnesium oxide, heavy | 6.60 |
| Croscannellose sodium | 10.00 |
| Ferric oxide red | 0.40 |
| Zinc stearate | 1.00 |
| Purified water | q.s.¹ |
| **Total first layer** | **200.00** |
| Tribuffer alkaline granules² | 199.00 |
| Zinc stearate | 1.00 |
| Total second (intermediate) layer | 200.00 |
| Aspirin 10% starch granules ³ | 361.10 mg in total (325 mg aspirin) |
| Zinc stearate | 1.50 |
| **Total third layer** | **362.00** |
| **Total tablet weight** | **762.00** |

| | |
|---|---|
| ¹ The purified water in the first layer in Example 1 (as well as in Examples 2 to 6 and 8 to 10) is used for wet granulation and removed during drying. ² The tribuffer alkaline granules used in the Example 1 formulation and in Examples 2 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. ³ The aspirin 10% starch granules used in the Example 1 formulation and in Examples 2 to 10 is obtained from Rhodia, Cranbury NJ, USA. | |

A wet granulation process is used to prepare pravastatin granules, dissolving the pravastatin sodium and the povidone in purified water and using this solution to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The triple layer tablets are prepared by having the granules of powder mixes for each layer in separate hoppers on a tablet press designed for the manufacture of triple layered tablets. Operating the tablet press results in triple layers tablets containing 81 mg of aspirin and 20 mg of pravastatin sodium possessing an intermediate layer containing alkaline earth buffers.

### Reference - EXAMPLE 10

A trilayered tablet containing 81mg of aspirin in the first outer layer and 40mg pravastatin sodium in a second outer layer and a non-buffered intermediate layer may be prepared as follows:

| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| Purified water | q.s.¹ |
| **Total first layer** | **400.00** |
| Lactose | 193.00 |
| Croscarmellose sodium | 6.00 |
| Zinc stearate | 1.00 |
| Aspirin 10% starch granules³ | 90.00 (81 mg aspirin) |
| Microcrystalline cellulose | 48.00 |
| Lactose | 160.50 |
| Zinc stearate | 1.50 |
| **Total third layer** | **300.00** |
| **Total tablet weight** | **900.00** |

| | |
|---|---|
| ¹ The purified water in the first layer in Example 1 (as well as in Examples 2 to 6 and 8 to 10) is used for wet granulation and removed during drying. ³ The aspirin 10% starch granules used in the Example 1 formulation and in Examples 2 to 10 is obtained from Rhodia, Cranbury NJ, USA. | |

A wet granulation process is used to prepare pravastatin granules, dissolving the pravastatin sodium and the povidone in purified water and using this solution to granulate the mixture of the other first layer ingredients except for the zinc stearate. The granules are dried, sieved and lubricated by mixing with the zinc stearate.

The second, intermediate layer components are mixed together dry in a suitable tumble blender.

The aspirin layer components, except for zinc stearate, are combined by mixing in a suitable tumble blender. The zinc stearate is then added to this mixture and mixing continued to lubricate the blend.

The triple layer tablets are prepared by having the granules of powder mixes for each layer in separate hoppers on a tablet press designed for the manufacture of layered tablets. Operating the tablet press results in triple layers tablets containing 81 mg of aspirin and 40 mg of pravastatin sodium possessing an unbuffered intermediate layer.

## Claims

1. A pharmaceutical composition comprising pravastatin and aspirin in a formulation designed to reduce pravastatin:aspirin interaction wherein the pravastatin and aspirin are formulated together in a sandwich structure, the aspirin being present in a first layer, and the pravastatin being present in another layer, and a second or middle barrier layer separating said first layer containing aspirin from said other layer containing pravastatin, wherein the middle barrier layer includes one or more buffering agents.

2. The composition as defined in Claim 1 Wherein said sandwich structure is in the form of a tablet.

3. The composition as defined in Claim 1 or 2 containing 20 mg, 40 mg, or 80 mg pravastatin.

4. The composition as defined in any one of Claims 1 to 3 containing 81 mg or 325 mg aspirin.

5. The composition as defined in Claim 1 wherein the first layer comprises aspirin granules, one or more bulking agents and optionally one or more lubricants and optionally one or more other excipients.

6. The composition as defined in Claim 1 wherein the other layer comprises pravastatin, one or more bulking agents, optionally one or more binders, optionally one or more buffering agents, optionally one or more lubricants, and optionally one or more other excipients.

7. The composition as defined in Claim 1 wherein the middle barrier layer further comprises one or more bulking agents, and optionally one or more other excipients.

8. The composition as defined in any one of Claims 1 to 7 wherein the buffering agent is calcium carbonate, magnesium oxide, magnesium carbonate, magnesium hydroxide, calcium hydroxide, sodium phosphate, aluminum hydroxide, dihydroxyaluminum sodium carbonate, sodium carbonate, sodium bicarbonate, aluminum magnesium hydroxide sulfate or aluminum hydroxide magnesium carbonate co-dried gel, sodium acetate, sodium citrate, sodium tartrate, sodium fumarate, sodium malate, sodium succinate, aluminum oxide, or mixtures of two or more thereof.

9. The composition as defined in any one of Claims 1 to 8 wherein the middle barrier layer is comprised of tribuffer alkaline granules comprised of calcium carbonate, magnesium oxide, magnesium carbonate, sodium phosphate monobasic, a bulking agent and citric acid, and optionally a tabletting lubricant.

10. The composition as defined in Claim 1 wherein the first layer compris es aspirin, one or more bulking agents, optionally one or more lubricants, and optionally one or more other excipients; the middle barrier layer comprises one or more bulking agents, one or more buffering agents, optionally one or more lubricants, and optionally one or more other excipients; and the other layer comprises pravastatin, one or more bulking agents, optionally one or more buffering agents, optionally one or more binders, optionally one or more disintegrating agents, optionally one or more lubricants, and optionally one or more other excipients.

11. The pharmaceutical composition as defined in Claim 2 further including an outer protective coating or finishing layer surrounding said tablet.

12. The pharmaceutical composition as defined in Claim 1 wherein the aspirin is in the form of enteric coated aspirin granules.

13. The pharmaceutical composition as defined in Claim 1 comprising trilayered tablet comprising an outer layer containing pravastatin, another outer layer containing aspirin and the middle barrier layer includes one or more buffering agents.

14. The pharmaceutical composition as defined in Claim 1 comprising a trilayered tablet.

15. The pharmaceutical composition as defined in Claim 1 in the form of a trilayered tablet having the following composition:
First Layer containing Aspirin
granules containing aspirin and starch
Optional Lactose
Optional Microcrystalline Cellulose
Optional Croscarmellose Sodium
Magnesium or Zinc Stearate
Middle Barrier Layer
Tribuffer Alkaline Granules having the composition:
calcium carbonate
magnesium carbonate
magnesium oxide
sodium phosphate monobasic
cornstarch
citric acid
Optional Microcrystalline Cellulose
Zinc or Magnesium Stearate
Third Layer containing Pravastatin
Pravastatin Sodium
Lactose
Microcrystalline Cellulose
Povidone
Magnesium Oxide
Croscarmellose Sodium
Magnesium or Zinc Stearate

16. The pharmaceutical composition as defined in Claim 1 in the form of a tablet having the following composition:
**TABLET 1**
| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| Purified water | q.s.¹ |
| **Total first layer** | **400.00** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules | 90 mg containing 81 mg aspirin |
| Microcrystalline cellulose | 48.00 |
| Lactose | 160.50 |
| Zinc stearate | 1.50 |
| **Total third layer** | **300.00** |
| **Total tablet weight** | **900.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 2**
| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Iron oxide red | 0.80 |
| Zinc stearate | 2.00 |
| Purified water¹ | q.s. |
| **Total first layer** | **400.00** |
| Tribuffer alkaline granules² | 199.00 |
| Zinc stearate | 1.00 |
| **Total second, intermediate layer** | **200.00** |
| Aspirin 10% starch granules | 361.10 mg containing 325 mg aspirin |
| Zinc stearate | 0.90 |
| **Total third layer** | **362.00** |
| **Total tablet weight** | **962.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 3**
| **Ingredient** | **Amount per tablet (mg)** |
|---|---|
| Pravastatin sodium | 80.00 |
| Lactose monohydrate | 211.80 |
| Microcrystalline cellulose | 60.20 |
| Povidone | 4.00 |
| Magnesium oxide, heavy | 28.00 |
| Croscarmellose sodium | 20.00 |
| Blue #2 aluminum lake 11-14% | 0.80 |
| Zinc stearate | 2.00 |
| Purified water | q.s.¹ |
| **Total first layer** | **406.80** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules | 90 mg containing 81 mg aspirin |
| Microcrystalline cellulose | 48.00 |
| Lactose | 160.50 |
| Zinc stearate | 1.50 |
| **Total third layer** | **300.00** |
| **Total tablet weight** | **906.80** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 4**
| **Ingredient** | **Amount per tablet (mg)** |
|---|---|
| Pravastatin sodium | 80.00 |
| Lactose monohydrate | 212.60 |
| Microcrystalline cellulose | . 60.20 |
| Povidone | 4.00 |
| Magnesium oxide, heavy | 28.00 |
| Crosearmellose sodium | 20.00 |
| Zinc stearate | 2.00 |
| Purified water¹ | q.s. |
| **Total first layer** | **406.80** |
| Tribuffer alkaline granules² | 199.00 |
| Zinc stearate | 1.00 |
| **Total second, intermediate layer** | **200.00** |
| Aspirin 10% starch granules | 361.10 mg containing 325 mg aspirin |
| Zinc stearate | 0.90 |
| **Total third layer** | **362.00** |
| **Total tablet weight** | **968.80** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 5**
| **Ingredient** | **Amount per tablet (mg)** |
|---|---|
| Pravastatin sodium | 40.00 |
| Lactose monohydrate | 263.00 |
| Microcrystalline cellulose | 60.70 |
| Povidone | 4.00 |
| Magnesium oxide, heavy | 13.30 |
| Croscarmellose sodium | 20.20 |
| Ferric oxide yellow | 0.80 |
| Zinc stearate | 3.00 |
| Purified water | q.s.¹ |
| **Total first layer** | **405.00** |
| Tribuffer alkaline granules² | 199.00 |
| Magnesium stearate | 1.00 |
| Total second (intermediate) layer | 200.00 |
| Aspirin 10% starch granules | 90.08 mg containing 81 mg aspirin |
| Croscarmellose sodium | 8.85 |
| Lactose | 193.86 |
| Zinc stearate | 2.21 |
| **Total third layer** | **295.00** |
| **Total tablet weight** | **900.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 6 or 7**
| **Ingredient** | **Amount per tablet (mg)** |
|---|---|
| Pravastatin sodium | 40.00 |
| Lactose monohydrate | 259.80 |
| Microcrystalline cellulose | 60.20 |
| Povidone | 4.00 |
| Magnesium oxide, heavy | 13.20 |
| Croscarmellose sodium | 20.00 |
| Ferric oxide yellow | 0.80 |
| Zinc stearate | 2.00 |
| Purified water | q.s.¹ |
| **Total first layer** | **400.00** |
| Tribuffer alkaline granules² | 198.84 |
| Brown dye PB-1596 | 0.16 |
| Magnesium stearate | 1.00 |
| Total second (intermediate) layer | 200.00 |
| Aspirin 10% starch granules | 89.55 mg containing 81 mg aspirin |
| Lactose Fast-Flo | 160.95 |
| Microcrystalline cellulose | 48.00 |
| Zinc stearate | 1.50 |
| **Total third layer** | **300.00** |
| **Total tablet weight.** | **900.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
wherein a wet granulation process is used to prepare pravastatin granules of Tablet 6 and a direct compression process is used to prepare the pravastatin layer of Tablet 7.
**TABLET 8**
| **Ingredient** | **Amount per tablet (mg)** |
|---|---|
| Pravastatin sodium | 20.00 |
| Lactose monohydrate | 129.90 |
| Microcrystalline cellulose | 30.10 |
| Povidone | 2.00 |
| Magnesium oxide, heavy | 6.60 |
| Croscarmellose sodium | 10.00 |
| Ferric oxide yellow | 0.40 |
| Zinc stearate | 1.00 |
| Purified water | q.s.¹ |
| **Total first layer** | **200.00** |
| Tribuffer alkaline granules² | 75.00 |
| Microcrystalline cellulose | 124.00 |
| Zinc stearate | 1.00 |
| **Total second (intermediate) layer** | **200.00** |
| Aspirin 10% starch granules | 90.00 mg containing 81 mg aspirin |
| Lactose Fast-Flo | 160.50 |
| Microcrystalline cellulose | 48.00 |
| Zinc stearate | 1.50 |
| T**otal third layer** | **300.00** |
| **Total tablet weight** | **700.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |
; or
**TABLET 9**
| **Ingredient** | **Amount per tablet (mg)** |
|---|---|
| Pravastatin sodium | 20.00 |
| Lactose monohydrate | 129.00 |
| Microcrystalline cellulose | 30.10 |
| Povidone | 2.00 |
| Magnesium oxide, heavy | 6.60 |
| Croscarmellose sodium | 10.00 |
| Ferric oxide red | 0.40 |
| Zinc stearate | 1.00 |
| Purified water | q.s.¹ |
| **Total first layer** | **200.00** |
| Tribuffer alkaline granules² | 199.00 |
| Zinc stearate | 1.00 |
| **Total second (intermediate) layer** | **200.00** |
| Aspirin 10% starch granules | 361.10 mg containing 325 mg aspirin |
| Zinc stearate | 1.50 |
| **Total third layer** | **362.00** |
| **Total tablet weight** | **762.00** |
| | |
|---|---|
| ¹ The purified water in the first layer in Tablets 1 to 6, 8 and 9 is used for wet granulation and removed during drying. ^{2.} The tribuffer alkaline granules used in Tablets 1 to 9 has the following composition: calcium carbonate 52.63%w/w, magnesium oxide heavy 21.05% w/w, magnesium carbonate 13.16% w/w, monobasic sodium phosphate 1.32 %w/w, cornstarch 10.52 % w/w, citric acid 1.32 % w/w. | |

17. The pharmaceutical composition as defined in Claim 2 wherein the aspirin is in the form of enteric coated granules of aspirin and/or the statin is in the form of enteric coated granules of statin.

18. A pharmaceutical composition as defined in any one of Claims 1 to 17 for lowering serum cholesterol or preventing or inhibiting or treating atherosclerosis or reducing risk of or treating a cardiovascular event or disease, coronary artery disease or cerebrovascular disease.

## Patentansprüche

1. Pharmazeutische Zusammensetzung, aufweisend Pravastatin und Aspirin in einer Formulierung, die zur Verringerung der Pravastatin/Aspirin-Wechselwirkung ausgelegt ist, wobei das Pravastatin und Aspirin gemeinsam in einer Sandwichstruktur formuliert sind und das Aspirin in einer ersten Lage vorliegt und das Pravastatin in einer anderen Lage vorliegt und eine zweite oder mittlere Sperrschicht die erste, das Aspirin enthaltende Lage von der anderen, das Pravastatin enthaltende Lage trennt, wobei die mittlere Sperrschicht ein oder mehrere Mittel zum Puffern einschließt.

2. Zusammensetzung nach Anspruch 1, wobei die Sandwichstruktur die Form einer Tablette annimmt.

3. Zusammensetzung nach Anspruch 1 oder 2, enthaltend 20 mg, 40 mg oder 80 mg Pravastatin.

4. Zusammensetzung nach einem der Ansprüche 1 bis 3, enthaltend 81 mg oder 325 mg Aspirin.

5. Zusammensetzung nach Anspruch 1, wobei die erste Lage Aspirin-Granalien aufweist, einen oder mehrere Füllstoffe und wahlweise ein oder mehrere Gleitmittel und wahlweise ein oder mehrere andere Hilfsstoffe.

6. Zusammensetzung nach Anspruch 1, wobei die andere Lage Pravastatin aufweist, einen oder mehrere Füllstoffe, wahlweise ein oder mehrere Bindemittel, wahlweise ein oder mehrere Mittel zum Puffern, wahlweise ein oder mehrere Gleitmittel und wahlweise einen oder mehrere andere Hilfsstoffe.

7. Zusammensetzung nach Anspruch 1, wobei die mittlere Sperrschicht ferner einen oder mehrere Füllstoffe aufweist und wahlweise ein oder mehrere andere Hilfsstoffe.

8. Zusammensetzung nach einem der Ansprüche 1 bis 7, wobei das Mittel zum Puffern Calciumcarbonat ist, Magnesiumoxid, Magnesiumcarbonat, Magnesiumhydroxid, Calciumhydroxid, Natriumphosphat, Aluminiumhydroxid, Dihydroxyaluminiumnatriumcarbonat, Natriumcarbonat, Natriumhydrogencarbonat, Aluminium-Magnesiumhydroxidsulfat oder Aluminiumhydroxid-Magnesiumcarbonat-co-getrocknetes Gel, Natriumacetat, Natriumcitrat, Natriumtartrat, Natriumfumarat, Natriummalat, Natriumsuccinat, Aluminiumoxid oder Mischungen von zwei oder mehreren davon.

9. Zusammensetzung nach einem der Ansprüche 1 bis 8, wobei die mittlere Sperrschicht aus Tripuffer-Alkaligranalien aufgebaut ist, die Calciumcarbonat aufweisen, Magnesiumoxid, Magnesiumcarbonat, Natriumdihydrogenphosphat, ein Füllmittel und Citronensäure, und wahlweise aus einem Gleitmittel zur Tablettierung.

10. Zusammensetzung nach Anspruch 1, wobei die erste Lage Aspirin aufweist, einen oder mehrere Füllstoffe, wahlweise ein oder mehrere Gleitmittel und wahlweise einen oder mehrere andere Hilfsstoffe; wobei die mittlere Sperrschicht einen oder mehrere Füllstoffe aufweist, ein oder mehrere Mittel zum Puffern, wahlweise ein oder mehrere Gleitmittel und wahlweise einen oder mehrere andere Hilfsstoffe; und wobei die andere Lage Pravastatin aufweist, einen oder mehrere Füllstoffe, wahlweise ein oder mehrere Mittel zum Puffern, wahlweise ein oder mehrere Bindemittel, wahlweise ein oder mehrere Zerfallhilfsmittel, wahlweise ein oder mehrere Gleitmittel und wahlweise einen oder mehrere andere Hilfsstoffe.

11. Pharmazeutische Zusammensetzung nach Anspruch 2, ferner einschließend eine äußere, schützende Schicht oder konfektionierende Lage, die die Tablette umgibt.

12. Pharmazeutische Zusammensetzung nach Anspruch 1, wobei das Aspirin in Form von magensaftresistent beschichteten Aspirin-Granalien vorliegt.

13. Pharmazeutische Zusammensetzung nach Anspruch 1, aufweisend eine dreilagige Tablette mit einer äußeren Lage, die Pravastatin enthält, einer anderen äußeren Lage, die Aspirin enthält, und der mittleren Sperrschicht, in die ein oder mehrere Mittel zum Puffern einbezogen sind.

14. Pharmazeutische Zusammensetzung nach Anspruch 1, die eine dreilagige Tablette umfasst.

15. Pharmazeutische Zusammensetzung nach Anspruch 1 in Form einer dreilagigen Tablette mit der folgenden Zusammensetzung:
Aspirin enthaltende erste Lage
Aspirin und Stärke enthaltende Granalien
wahlweise Lactose
wahlweise mikrokristalline Cellulose
wahlweise Natrium-Croscarmellose
Magnesium- oder Zinkstearat
Mittlere Sperrschicht
Tripuffer-Alkaligranalien mit der Zusammensetzung:
Calciumcarbonat
Magnesiumcarbonat
Magnesiumoxid
Natriumdihydrogenphosphat
Maisstärke
Citronensäure
wahlweise mikrokristalline Cellulose
Zink- oder Magnesiumstearat
Pravastatin enthaltende dritte Lage
Pravastatin-Natrium
Lactose
mikrokristalline Cellulose
Povidon
Magnesiumoxid
Natrium-Croscarmellose
Magnesium- oder Zinkstearat

16. Pharmazeutische Zusammensetzung nach Anspruch 1 in Form einer Tablette mit der folgenden Zusammensetzung:
**TABLETTE 1**
| Inhaltsstoff | Menge pro Tablette (mg) |
|---|---|
| Pravastatin-Natrium | 40,00 |
| Lactosemonohydrat | 259,80 |
| mikrokristalline Cellulose | 60,20 |
| Povidon | 4,00 |
| Magnesiumoxid, schwer | 13,20 |
| Natrium-Croscarmellose | 20,00 |
| Eisenoxidgelb | 0,80 |
| Zinkstearat | 2,00 |
| gereinigtes Wasser | q.s.¹ |
| Summe der ersten Lage | 400,00 |
| Tripuffer-Alkaligranalien² | 75,00 |
| mikrokristalline Cellulose | 124,00 |
| Zinkstearat | 1,00 |
| Summe zweite Lage (mittlere) | 200,00 |
| Aspirin-10%Stärkegranalien | 90 mg mit 81 mg Aspirin |
| mikrokristalline Zellulose | 48,00 |
| Lactose | 160,50 |
| Zinkstearat | 1,50 |
| Summe dritte Lage | 300,00 |
| Summe: Tablettenmasse | 900,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 2**
| Inhaltsstoff | Menge pro Tablette (mg) |
|---|---|
| Pravastatin-Natrium | 40,00 |
| Lactosemonohydrat | 259,80 |
| mikrokristalline Cellulose | 60,20 |
| Povidon | 4,00 |
| Magnesiumoxid, schwer | 13,20 |
| Natrium-Croscarmellose | 20,00 |
| Eisenoxidrot | 0,80 |
| Zinkstearat | 2,00 |
| gereinigtes Wasser¹ | q.s. |
| Summe erste Lage | 400,00 |
| Tripuffer-Alkligranalien² | 199,00 |
| Zinkstearat | 1,00 |
| Summe zweite Lage (mittlere) | 200,00 |
| Aspirin-10%Stärkegranalien | 361,10 mg mit 325 mg Aspirin |
| Zinkstearat | 0,90 |
| Summe dritte Lage | 362,00 |
| Summe Tablettenmasse | 962,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 3**
| Inhaltsstoff | Menge pro Tablette (mg) |
|---|---|
| Pravastatin-Natrium | 80,00 |
| Lactosemonohydrat | 211,80 |
| mikrokristalline Cellulose | 60,20 |
| Povidon | 4,00 |
| Magnesiumoxid, schwer | 28,00 |
| Natrium-Croscarmellose | 20,00 |
| Blau #2 Aluminiumplättchen 11-14% | 0,80 |
| Zinkstearat | 2,00 |
| gereinigtes Wasser | q.s.¹ |
| Summe erste Lage | 406,80 |
| Tripuffer-Alkaligranalien² | 75,00 |
| mikrokristalline Cellulose | 124,00 |
| Zinkstearat | 1,00 |
| Summe zweite Lage (mittlere) | 200,00 |
| Aspirin-10%Stärkegranalien | 90 mg mit 81 mg Aspirin |
| mikrokristalline Cellulose | 48,00 |
| Lactose | 160,50 |
| Zinkstearat | 1,50 |
| Summe dritte Lage | 300,00 |
| Summe Tablettenmasse | 906,80 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 4**
| Inhaltsstoff | Menge pro Tablette (mg) |
|---|---|
| Pravastatin-Natrium | 80,00 |
| Lactosemonohydrat | 212,60 |
| mikrokristalline Cellulose | 60,20 |
| Povidon | 4,00 |
| Magnesiumoxid, schwer | 28,00 |
| Natrium-Croscarmellose | 20,00 |
| Zinkstearat | 2,00 |
| gereinigtes Wasser¹ | q.s. |
| Summe erste Lage | 406,80 |
| Tripuffer-Alkaligranalien² | 199,00 |
| Zinkstearat | 1,00 |
| Summe zweite Lage (mittlere) | 200,00 |
| Aspirin-10%Stärkegranalien | 361,10 mg mit 325 mg Aspirin |
| Zinkstearat | 0,90 |
| Summe dritte Lage | 362,00 |
| Summe Tablettenmasse | 968,80 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 5**
| Inhaltsstoff | Menge pro Tablette (mg) |
|---|---|
| Pravastatin-Natrium | 40,00 |
| Lactosemonohydrat | 263,00 |
| mikrokristalline Cellulose | 60,70 |
| Povidon | 4,00 |
| Magnesiumoxid, schwer | 13,30 |
| Natrium-Croscarmellose | 20,20 |
| Eisenoxidgelb | 0,80 |
| Zinkstearat | 3,00 |
| gereinigtes Wasser | q.s.¹ |
| Summe erste Lage | 405,00 |
| Tripuffer-Alkaligranalien² | 199,00 |
| Magnesiumstearat | 1,00 |
| Summe zweite Lage (mittlere) | 200,00 |
| Aspirin-10%Stärkegranalien | 90,08 mg mit 81 mg Aspirin |
| Natrium-Croscarmellose | 8,85 |
| Lactose | 193,86 |
| Zinkstearat | 2,21 |
| Summe dritte Lage | 295,00 |
| Summe Tablettenmasse | 900,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 6 ODER 7**
| Inhaltsstoff | Menge pro Tablette (mg) |
|---|---|
| Pravastatin-Natrium | 40,00 |
| Lactosemonohydrat | 259,80 |
| mikrokristalline Cellulose | 60,20 |
| Povidon | 4,00 |
| Magnesiumoxid, schwer | 13,20 |
| Natrium-Croscarmellose | 20,00 |
| Eisenoxidgelb | 0,80 |
| Zinkstearat | 2,00 |
| gereinigtes Wasser | q.s.¹ |
| Summe erste Lage | 400,00 |
| Tripuffer-Alkaligranalien² | 198,84 |
| brauner Farbstoff PB-1596 | 0,16 |
| Magnesiumstearat | 1,00 |
| Summe zweite Lage (mittlere) | 200,00 |
| Aspirin-10%Stärkegranalien | 89,55 mg mit 81 mg Aspirin |
| Lactose Fast-Flo | 160,95 |
| mikrokristalline Cellulose | 48,00 |
| Zinkstearat | 1,50 |
| Summe dritte Lage | 300,00 |
| Summe Tablettenmasse | 900,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
worin ein Prozess der Feuchtgranulierung zur Anwendung gelangt, um Pravastatin-Granalien der Tabletten 6 herzustellen, sowie ein direkter Pressprozess, der zur Herstellung der Pravastatin-Lage der Tablette 7 angewendet wird;
**TABLETTE 8**
| Inhaltsstoff | Menge pro Tablette (mg) |
|---|---|
| Pravastatin-Natrium | 20,00 |
| Lactosemonohydrat | 129,90 |
| mikrokristalline Cellulose | 30,10 |
| Povidon | 2,00 |
| Magnesiumoxid, schwer | 6,60 |
| Natrium-Croscarmellose | 10,00 |
| Eisenoxidgelb | 0,40 |
| Zinkstearat | 1,00 |
| gereinigtes Wasser | q.s.¹ |
| Summe erste Lage | 200,00 |
| Tripuffer-Alkaligranalien² | 75,00 |
| mikrokristalline Cellulose | 124,00 |
| Zinkstearat | 1,00 |
| Summe zweite Lage (mittlere) | 200,00 |
| Aspirin-10%Stärkegranalien | 90,00 mg mit 81 mg Aspirin |
| Lactose Fast-Flo | 160,50 |
| mikrokristalline Cellulose | 48,00 |
| Zinkstearat | 1,50 |
| Summe dritte Lage | 300,00 |
| Summe Tablettenmasse | 700,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |
oder
**TABLETTE 9**
| Inhaltsstoff | Menge pro Tablette (mg) |
|---|---|
| Pravastatin-Natrium | 20,00 |
| Lactosemonohydrat | 129,00 |
| mikrokristalline Cellulose | 30,10 |
| Povidon | 2,00 |
| Magnesiumoxid, schwer | 6,60 |
| Natrium-Croscarmellose | 10,00 |
| Eisenoxidrot | 0,40 |
| Zinkstearat | 1,00 |
| gereinigtes Wasser | q.s.¹ |
| Summe erste Lage | 200,00 |
| Tripuffer-Alkaligranalien² | 199,00 |
| Zinkstearat | 1,00 |
| Summe zweite Lage (mittlere) | 200,00 |
| Aspirin. 10%Stärkegranalien | 361,10 mg mit 325 mg Aspirin |
| Zinkstearat | 1,50 |
| Summe dritte Lage | 362,00 |
| Summe Tablettenmasse | 762,00 |
| | |
|---|---|
| ¹ Das gereinigte Wasser in der ersten Lage in den Tabletten 1 bis 6 und 8 und 9 wird bei der Feuchtgranulierung angewendet und während des Trocknens entfernt. ² Die Tripuffer-Alkaligranalien, die in den Tabletten 1 bis 9 verwendet werden, haben die folgende Zusammensetzung: Calciumcarbonat 52,63% (Gewicht/Gewicht), Magnesiumoxid, schwer 21,05% (Gewicht/Gewicht), Magnesiumcarbonat 13,16% (Gewicht/Gewicht), Natriumdihydrogenphosphat 1,32% (Gewicht/Gewicht), Maisstärke 10,52% (Gewicht/Gewicht), Citronensäure 1,32% (Gewicht/Gewicht). | |

17. Pharmazeutische Zusammensetzung nach Anspruch 2, wobei das Aspirin in Form magensaftresistent beschichteter Granalien von Aspirin und oder der Statin in Form vom magensaftresistent beschichteten Granalien von Statin vorliegt.

18. Pharmazeutische Zusammensetzung nach einem der Ansprüche 1 bis 17 zur Senkung des Serum-Cholesterins oder Verhütung oder Hemmung oder Behandlung von Atherosclerose oder Verringerung des Risikos oder Behandlung eines kardiovaskulären Ereignisses oder Erkrankung, einer koronaren Herzkrankheit oder zerebrovaskulären Erkrankung.

## Revendications

1. Composition pharmaceutique comprenant de la pravastatine et de l'aspirine dans une formulation conçue pour réduire l'interaction pravastatine:aspirine, dans laquelle la pravastatine et l'aspirine sont formulées ensemble dans une structure en sandwich, l'aspirine étant présente dans une première couche, et la pravastatine étant présente dans une autre couche, et une seconde couche ou couche barrière du milieu séparant ladite première couche contenant l'aspirine de ladite autre couche contenant la pravastatine, où la couche barrière du milieu inclut un ou plusieurs agents tampons.

2. Composition selon la revendication 1 dans laquelle ladite structure en sandwich est sous la forme d'un comprimé.

3. Composition selon la revendication 1 ou 2 contenant 20 mg, 40 mg, ou 80 mg de pravastatine.

4. Composition selon l'une quelconque des revendications 1 à 3 contenant 81 mg ou 325 mg d'aspirine.

5. Composition selon la revendication 1 dans laquelle la première couche comprend des granules d'aspirine, un ou plusieurs agents gonflants et éventuellement un ou plusieurs lubrifiants et éventuellement un ou plusieurs autres excipients.

6. Composition selon la revendication 1 dans laquelle l'autre couche comprend de la pravastatine, un ou plusieurs agents gonflants, éventuellement un ou plusieurs liants, éventuellement un ou plusieurs agents tampons, éventuellement un ou plusieurs lubrifiants, et éventuellement un ou plusieurs autres excipients.

7. Composition selon la revendication 1 dans laquelle la couche barrière du milieu comprend en outre un ou plusieurs agents gonflants, et éventuellement un ou plusieurs autres excipients.

8. Composition selon l'une quelconque des revendications 1 à 7 dans laquelle l'agent tampon est le carbonate de calcium, l'oxyde de magnésium, le carbonate de magnésium, l'hydroxyde de magnésium, l'hydroxyde de calcium, le phosphate de sodium, l'hydroxyde d'aluminium, le carbonate de dihydroxyaluminium et de sodium, le carbonate de sodium, le bicarbonate de sodium, un gel co-déshydraté d'hydroxyde-sulfate d'aluminium-magnésium ou d'hydroxyde d'aluminium et de carbonate de magnésium, l'acétate de sodium, le citrate de sodium, le tartrate de sodium, le fumarate de sodium, le malate de sodium, le succinate de sodium, l'oxyde d'aluminium, ou les mélanges de deux ou plusieurs d'entre eux.

9. Composition selon l'une quelconque des revendications 1 à 8 dans laquelle la couche barrière du milieu consiste en granules alcalins tritampons composés de carbonate de calcium, d'oxyde de magnésium, de carbonate de magnésium, de phosphate de sodium monobasique, d'un agent gonflant et d'acide citrique, et éventuellement en un lubrifiant de comprimé.

10. Composition selon la revendication 1 dans laquelle la première couche comprend de l'aspirine, un ou plusieurs agents gonflants, éventuellement un ou plusieurs lubrifiants, et éventuellement un ou plusieurs autres excipients; la couche barrière du milieu comprend un ou plusieurs agents gonflants, un ou plusieurs agents tampons, éventuellement un ou plusieurs lubrifiants, et éventuellement un ou plusieurs autres excipients; et l'autre couche comprend de la pravastatine, un ou plusieurs agents gonflants, éventuellement un ou plusieurs agents tampons, éventuellement un ou plusieurs liants, éventuellement un ou plusieurs agents désintégrants, éventuellement un ou plusieurs lubrifiants, et éventuellement un ou plusieurs autres excipients.

11. Composition pharmaceutique selon la revendication 2 incluant en outre une couche de revêtement
ou de finissage protectrice extérieure entourant ledit comprimé.

12. Composition pharmaceutique selon la revendication 1 dans laquelle l'aspirine est sous la forme de granules d'aspirine à enrobage entérique.

13. Composition pharmaceutique selon la revendication 1 comprenant un comprimé tricouche comprenant une couche extérieure contenant de la pravastatine, une autre couche extérieure contenant de l'aspirine et la couche barrière du milieu inclut un ou plusieurs agents tampons.

14. Composition pharmaceutique selon la revendication 1 comprenant un comprimé tricouche.

15. Composition pharmaceutique selon la revendication 1 sous la forme d'un comprimé tricouche ayant la composition suivante:
PREMIERE COUCHE CONTENANT DE L'ASPIRINE
Granules contenant de l'aspirine et de l'amidon
Lactose optionnel
Cellulose microcristalline optionnelle
Croscarmellose sodique optionnelle
Stéarate de magnésium ou de zinc
COUCHE BARRIERE DU MILIEU
Granules alcalins tritampons ayant comme composition:
carbonate de calcium
carbonate de magnésium
oxyde de magnésium
phosphate de sodium monobasique
amidon de maïs
acide citrique,
Cellulose microcristalline optionnelle
Stéarate de magnésium ou de zinc
TROISIEME COUCHE CONTENANT DE LA PRAVASTATINE
Pravastatine sodique
Lactose
Cellulose microcristalline
Povidone
Oxyde de magnésium
Croscarmellose sodique
Stéarate de magnésium ou de zinc

16. Composition pharmaceutique selon la revendication 1 sous la forme d'un comprimé ayant la composition suivante:
**COMPRIME 1**
| Ingrédient | Quantité par comprimé (mg) |
|---|---|
| Pravastatine sodique | 40,00 |
| Lactose monohydrate | 259,80 |
| Cellulose microcristalline | 60,20 |
| Povidone | 4,00 |
| Oxyde de magnésium, lourd | 13,20 |
| Croscarmellose sodique | 20,00 |
| Oxyde ferrique jaune | 0,80 |
| Stéarate de zinc | 2,00 |
| Eau purifiée | q.s.¹ |
| Total première couche | 400,00 |
| Granules alcalins tritampons² | 75,00 |
| Cellulose microcristalline | 124,00 |
| Stéarate de zinc | 1,00 |
| Total seconde couche (intermédiaire) | 200,00 |
| Granules d'aspirine-10% d'amidon | 90 mg contenant 81 mg d'aspirine |
| Cellulose microcristalline | 48,00 |
| Lactose | 160,50 |
| Stéarate de zinc | 1,50 |
| Total troisième couche | 300,00 |
| Poids comprimé total | 900,00 |
ou
**COMPRIME 2**
| Ingrédient | Quantité par comprimé (mg) |
|---|---|
| Pravastatine sodique | 40,00 |
| Lactose monohydrate | 259,80 |
| Cellulose microcristalline | 60,20 |
| Povidone | 4,00 |
| Oxyde de magnésium, lourd | 13,20 |
| Croscarmellose sodique | 20,00 |
| Oxyde de fer rouge | 0,80 |
| Stéarate de zinc | 2,00 |
| Eau purifiée¹ | q.s. |
| Total première couche | 400,00 |
| Granules alcalins tritampons² | 199,00 |
| Stéarate de zinc | 1,00 |
| Total seconde couche intermédiaire | 200,00 |
| Granules d'aspirine-10% d'amidon | 361,10 mg contenant 325 mg d'aspirine |
| Stéarate de zinc | 0,90 |
| Total troisième couche | 362,00 |
| Poids comprimé total | 962,00 |
ou
**COMPRIME 3**
| Ingrédient | Quantité par comprimé (mg) |
|---|---|
| Pravastatine sodique | 80,00 |
| Lactose monohydrate | 211,80 |
| Cellulose microcristalline | 60,20 |
| Povidone | 4,00 |
| Oxyde de magnésium, lourd | 28,00 |
| Croscarmellose sodique | 20,00 |
| Laque d'aluminium Blue #2 11-14% | 0,80 |
| Stéarate de zinc | 2,00 |
| Eau purifiée | q.s.¹ |
| Total première couche | 406,80 |
| Granules alcalins tritampons² | 75,00 |
| Cellulose microcristalline | 124,00 |
| Stéarate de zinc | 1,00 |
| Total seconde couche (intermédiaire) | 200,00 |
| Granules d'aspirine-10% d'amidon | 90 mg contenant 81 mg d'aspirine |
| Cellulose microcristalline | 48,00 |
| Lactose | 160,50 |
| Stéarate de zinc | 1,50 |
| Total troisième couche | 300,00 |
| Poids comprimé total | 906,80 |
ou
**COMPRIME 4**
| Ingrédient | Quantité par comprimé (mg) |
|---|---|
| Pravastatine sodique | 80,00 |
| Lactose monohydrate | 212,60 |
| Cellulose microcristalline | 60,20 |
| Povidone | 4,00 |
| Oxyde de magnésium, lourd | 28,00 |
| Croscarmellose sodique | 20,00 |
| Stéarate de zinc | 2,00 |
| Eau purifiée¹ | q.s. |
| Total première couche | 406,80 |
| Granules alcalins tritampons² | 199,00 |
| Stéarate de zinc | 1,00 |
| Total seconde couche intermédiaire | 200,00 |
| Granules d'aspirine-10% d'amidon | 361,10 mg contenant 325 mg d'aspirine |
| Stéarate de zinc | 0,90 |
| Total troisième couche | 362,00 |
| Poids comprimé total | 968,80. |
ou
**COMPRIME 5**
| Ingrédient | Quantité par comprimé (mg) |
|---|---|
| Pravastatine sodique | 40,00 |
| Lactose monohydrate | 263,00 |
| Cellulose microcristalline | 60,70 |
| Povidone | 4,00 |
| Oxyde de magnésium, lourd | 13,30 |
| Croscarmellose sodique | 20,20 |
| Oxyde ferrique jaune | 0,80 |
| Stéarate de zinc | 3,00 |
| Eau purifiée | q.s.¹ |
| Total première couche | 405,00 |
| Granules alcalins tritampons² | 199,00 |
| Stéarate de magnésium | 1,00 |
| Total seconde couche (intermédiaire) | 200,00 |
| Granules d'aspirine-10% d'amidon | 90,08 mg contenant 81 mg d'aspirine |
| Croscarmellose sodique | 8,85 |
| Lactose | 193,86 |
| Stéarate de zinc | 2,21 |
| Total troisième couche | 295,00 |
| Poids comprimé total | 900,00 |
ou
**COMPRIME 6 ou 7**
| Ingrédient | Quantité par comprimé (mg) |
|---|---|
| Pravastatine sodique | 40,00 |
| Lactose monohydrate | 259,80 |
| Cellulose microcristalline | 60,20 |
| Povidone | 4,00 |
| Oxyde de magnésium, lourd | 13,20 |
| Croscarmellose sodique | 20,00 |
| Oxyde ferrique jaune | 0,80 |
| Stéarate de zinc | 2,00 |
| Eau purifiée | q.s.¹ |
| Total première couche | 400,00 |
| Granules alcalins tritampons² | 198,84 |
| Colorant brun PB-1596 | 0,16 |
| Stéarate de magnésium | 1,00 |
| Total seconde couche (intermédiaire) | 200,00 |
| Granules d'aspirine-10% d'amidon | 89,55 mg contenant 81 mg d'aspirine |
| Lactose Fast-Flo | 160,95 |
| Cellulose microcristalline | 48,00 |
| Stéarate de zinc | 1,50 |
| Total troisième couche | 300,00 |
| Poids comprimé total | 900,00 |
dans lequel un procédé de granulation par voie humide est utilisé pour préparer les granules de pravastatine du comprimé 6 et un procédé de compression directe est utilisé pour préparer la couche de pravastatine du comprimé 7,
ou
**COMPRIME 8**
| Ingrédient | Quantité par comprimé (mg) |
|---|---|
| Pravastatine sodique | 20,00 |
| Lactose monohydrate | 129,90 |
| Cellulose microcristalline | 30,10 |
| Povidone | 2,00 |
| Oxyde de magnésium, lourd | 6,60 |
| Croscarmellose sodique | 10,00 |
| Oxyde ferrique jaune | 0,40 |
| Stéarate de zinc | 1,00 |
| Eau purifiée | q.s. ¹ |
| Total première couche | 200,00 |
| Granules alcalins tritampons² | 75,00 |
| Cellulose microcristalline | 124,00 |
| Stéarate de zinc | 1,00 |
| Total seconde couche (intermédiaire) | 200,00 |
| Granules d'aspirine-10% d'amidon | 90,00 mg contenant 81 mg d'aspirine |
| Lactose Fast-Flo | 160,50 |
| Cellulose microcristalline | 48,00 |
| Stéarate de zinc | 1,50 |
| Total troisième couche | 300,00 |
| Poids comprimé total | 700,00 |
ou
**COMPRIME 9**
| Ingrédient | Quantité par comprimé (mg) |
|---|---|
| Pravastatine sodique | 20,00 |
| Lactose monohydrate | 129,00 |
| Cellulose microcristalline | 30,10 |
| Povidone | 2,00 |
| Oxyde de magnésium, lourd | 6,60 |
| Croscarmellose sodique | 10,00 |
| Oxyde ferrique rouge | 0,40 |
| Stéarate de zinc | 1,00 |
| Eau purifiée | q.s.¹ |
| Total première couche | 200,00 |
| Granules alcalins tritampons² | 199,00 |
| Stéarate de zinc | 1,00 |
| Total seconde couche (intermédiaire) | 200,00 |
| Granules d'aspirine-10% d'amidon | 361,10 mg contenant 325 mg d'aspirine |
| Stéarate de zinc | 1,50 |
| Total troisième couche | 362,00 |
| Poids comprimé total | 762,00 |
| | |
|---|---|
| ¹L'eau purifiée dans la première couche dans les comprimés 1 à 6, 8 et 9 est utilisée pour la granulation par voie humide et éliminée pendant le séchage. ²Les granules alcalins tritampons utilisés dans les comprimés 1 à 9 ont la composition suivante: carbonate de calcium 52,63% p/p, oxyde de magnésium lourd 21,05% p/p, carbonate de magnésium 13,16% p/p, phosphate de sodium monobasique 1,32% p/p, amidon de maïs 10,52% p/p, acide citrique 1,32% p/p. | |

17. Composition pharmaceutique selon la revendication 2 dans laquelle l'aspirine est sous forme de granules d'aspirine à enrobage entérique et/ou la statine est sous forme de granules de statine à enrobage entérique.

18. Composition pharmaceutique selon l'une quelconque des revendications 1 à 17 pour diminuer le cholestérol sérique ou prévenir ou inhiber ou traiter l'athérosclérose ou réduire le risque de ou traiter une maladie ou un événement cardiovasculaire, une maladie coronarienne ou une maladie cérébrovasculaire.
